# EUROPEAN PATENT APPLICATION

(11) **EP 1 430 890 A1**
(43) Date of publication of application: **23.06.2004**
(21) Application number: 02772928.4
(22) Date of filing: 26.09.2002
(51) Int. Cl.: A61K 31/138, A61P 7/02, A61P 9/00, A61P 9/10

(54) **THROMBUS/THROMBOGENESIS INHIBITORS**

(30) Priority: 26.09.2001 JP 2001292855
(71) Applicant: Mitsubishi Pharma Corporation, Osaka-shi, Osaka 541-0046 (JP)
(72) Inventor: KOBAYASHI, Yoshikazu, MITSUBISHI PHARMA CORP., Tokyo 103-8405 (JP); AZUMA, Hiroshi, MITSUBISHI PHARMA CORPORATION, Tokyo 103-8405 (JP); MOURI, Yuko, MITSUBISHI PHARMA CORPORATION, Tokyo 103-8405 (JP)
(74) Representative: von Kreisler, Alek, Dipl.-Chem.
(86) International application number: PCT/JP2002/009937
(87) International publication number: WO 2003/026636

(57) **Abstract**

The inhibitors of thrombus/thrombogenesis comprising as the active ingredient aminoalkoxybibenzyls represented by the following formula (I), pharmaceutically acceptable salts thereof or solvates thereof. 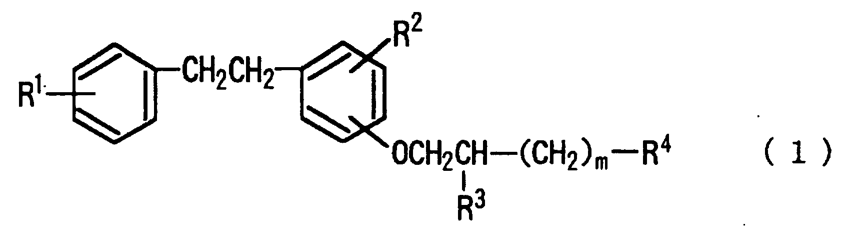 wherein R¹, R² and R³ represent each hydrogen, etc.; R⁴ represents -N(R⁵)(R⁶); and m is an integer of from 0 to 5. The above drugs are particularly efficacious for inhibiting thrombus/thrombogenesis in ischemic cerebrovascular failures selected from transient cerebral ischemic attack and cerebral infaction.

## Description

### TECHNICAL FIELD

This invention relates to a thrombus/thrombogenesis inhibitor.

### BACKGROUND ART

It is known that aminopropoxybibenzyls having specific structures typified by sarpogrelate hydrochloride represented by the following formula (2): 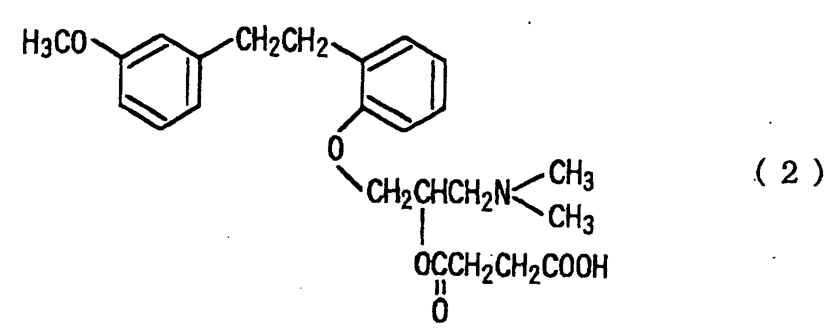 are effective as serotonin antagonists for improving various microcirculation disorders caused by thrombolic or vasoconstriction found in disorders of cerebral circulation failures, ischemic heart diseases or peripheral circulation failures and the like (Japanese Patent Unexamined Publication (Kokai) No. Hei 2-304022). However, it has not been reported that these compounds are useful for the inhibition of thrombus/thrombogenesis, particularly transient cerebral ischemic attack (optionally hereinafter referred to as "TIA") as far as the present inventors know.

On the contrary, clopidogrel as the platelet antiaggregation agent is effective for preventing the recurrence of ischemic cerebrovascular failures (U.S. Patent No. 5576328). However, while there have been many reports on the preventive effects on the onset of ischemic cerebrovascular failures by platelet antiaggregation agents, BMJ, 308, 81, 1994 reported that the reduction ratio of odds concerning stroke, myocardial infarction and vascular death against the control group in 142 trials is 27% (aspirin administration group: 25%; ticlopidine administration group: 33%; combination group of aspirin and ticlopidine: 28% and the like) in the whole group of administration of platelet antiaggregation agents, and among them, the reduction ratio of odds concerning non-fatal stroke against the control group in 18 trials performed subject to TIA patients or stroke patients is 23% according to Meta-Analysis by Antiplatelet Trialist (APT) Collaboration. Consequently, there is a limit to the efficacy (ratio of risk reduction) of the platelet antiaggregation agents against the prevention of onset (recurrence) of ischemic cerebrovascular failures.

It is reported that sarpogrelate hydrochloride binds selectively 5-HT2 serotonergic receptor (sometimes hereinafter referred to as receptor) in platelet and vascular smooth muscle, inhibits platelet aggregation, particularly platelet aggregation induced by serotonin (Thromb. Haemostas., 65, 415, 1991 and Jpn. Pharmacol. Ther., 19, S-593, 1991), inhibits serotonin-mediated vasoconstriction and vasoconstriction accompanied with platelet aggregation (Jpn. Pharmacol. Ther., 19, S-611, 1991), exhibits the improving effect on the deformability of red blood (Clinical Hemorheology, 12, 267, 1992) and exhibits improving effect peripheral circulation (J. Clin. Therap. Med., 7, 1205, 1991 and J. Clin. Therap. Med., 7, 1747, 1991).

Namely, sarpogrelate hydrochloride can be expected to exhibit the inhibitory effect against thrombus/thrombogenesis in the ischemic cerebrovascular failures selected from TIA and cerebral infarction by the different mode of action compared to aspirin exhibiting cyclooxygenase inhibitory activity as the main mode of action, and ticlopidine or clopidogrel exhibiting inhibitory activity against the suppression of adenylate cyclase or activating activity of adenylate cyclase as the main mode of action.

Consequently, the purpose of the present invention is to provide to new drugs for inhibiting thrombus/thrombogenesis.

### DISCLOSURE OF THE INVENTION

As a result that the present inventors have made intensive investigations to solve the aforementioned problems, they have found that aminopropoxybibenzyls having specific structures known as 5-HT2 receptor antagonists are useful for inhibiting thrombus/thrombogenesis, especially efficacious for inhibiting thrombus/thrombogenesis in ischemic cerebrovascular failures such as TIA and cerebral infarction and the like, and completed the present invention.

Namely, the gist of the present lies in inhibitors of thrombus/thrombogenesis comprising as the active ingredient aminoalkoxybibenzyls represented by the following formula (1), pharmaceutically acceptable salts thereof and solvates thereof: 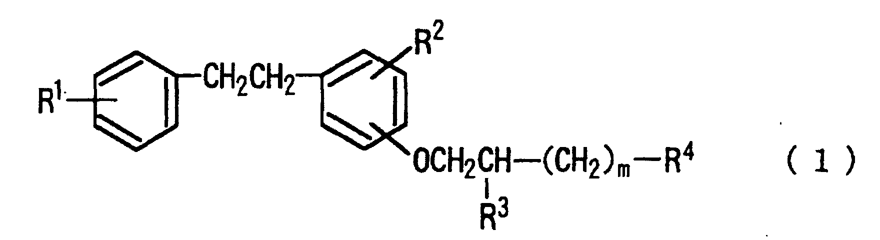 wherein R¹ represents hydrogen atom, a halogen atom, a C₁ to C₅ alkoxy group or a C₂ to C₆ dialkylamino group; R² represents hydrogen atom, a halogen atom or a C₁ to C₅ alkoxy group; R³ represents hydrogen atom, hydroxy group, -O-(CH₂)ₙ-COOH, wherein n represents an integer of from 1 to 5, or -O-CO-(CH₂)ₗ-COOH, wherein l represents an integer of from 1 to 3; R⁴ represents -N(R⁵)(R⁶), wherein R⁵ and R⁶ represent each independently hydrogen atom or a C₁ to C₈ alkyl group; or 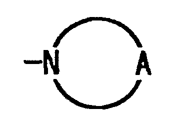 wherein A is a C₃ to C₅ alkylene group which may be substituted by carboxyl group; m represents an integer of from 0 to 5.

As the preferably embodiment of the present invention, there includes that the compounds represented by the formula (1) is selected from the compounds represented by the following formula (2), salts thereof and solvates thereof: 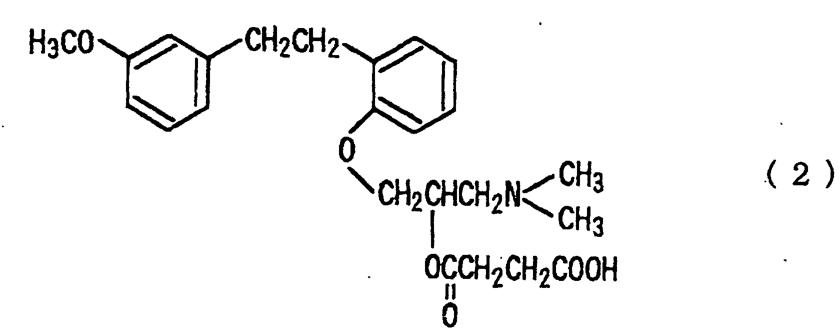 that the salt is hydrochloride; that the compounds represented by the formula (1) is selected from the compounds represented by the following formula (3), salts thereof and solvates thereof: 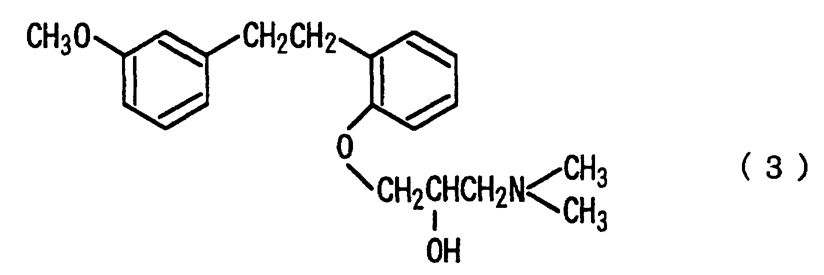 that inhibition of thrombus/thrombogenesis is the inhibition of thrombus/thrombogenesis in the diseases selected from angina pectoris, myocardial infaction and ischemic cerebrovascular failures; that the ischemic cerebrovascular failures are selected from transient cerebral ischemic attack and cerebral infarction; that angina pectoris is selected from transient chronic stable angina pectoris and unstable angina pectoris; that inhibitors of the present invention are combinedly administered to the patients already administered the drugs selected from platelet antiaggregation agents, anticoagulants and brain circulation metabolic stimulants; and that the inhibitors are administered to the patients administered the drugs selected from platelet antiaggregation agents, anticoagulants and cerebral circulation metabolism improving agents after discontinuation of the administration of said drugs.

Further, the gist of the present invention lies in improving agents for intermittent claudication which comprise administering drugs containing as the active ingredient aminoalkoxybibenzyls represented by the aforementioned formula (1), pharmaceutically acceptable salts thereof and solvates thereof and drugs selected from platelet antiaggregation agents other than the aforementioned drugs, anticoagulants and cerebral circulation metabolism improving agents. The preferable embodiments include that the compounds represented by the aforementioned formula (1) is the compound selected from the compound represented by the aforementioned formula (2), salts thereof and solvates thereof; that the salt is hydrochloride; that the compound represented by the aforementioned formula (1) is the compound selected the compounds represented by the aforementioned formula (3), salts thereof and solvates thereof; that the platelet antiaggregation agent is aspirin formulations or ticlopidine hydrochloride; and that the anticoagulant is warfarin potassium or urokinase

### BEST MODE FOR CARRYING OUT THE INVENTION

The present invention will be explained in more detail as follows:

The inhibitors of thrombus/thrombogenesis comprise as the active ingredient the aminoalkoxybibenzyls represented by the aforementioned formula (1) and pharmaceutically acceptable salts and solvates thereof. The salts encompass the broad meanings including esters.

It is known that the compounds represented by the aforementioned formula (1) are effective for the improvement of various microcirculation disorders caused by thrombogenesis or vasoconstriction found in disorders of cerebral circulation failures, ischemic heart diseases or peripheral circulation failures and the like (Japanese Patent Unexamined Publication (Kokai) No. Hei 2-304022). However, it is surprisingly unexpected results that the compounds are useful for the inhibition of thrombus/thrombogenesis, particularly the inhibition of thrombus/thrombogenesis found in ischemic cerebrovascular failures such as TIA and cerebral infarction and the like.

The aforementioned formula (1) will further explained in more detail. In the formula, R¹ represents hydrogen atom; a halogen atom such as chlorine atom, fluorine atom and the like; a C₁ to C₅ alkoxy group such as methoxy group, ethoxy group, butoxy group and the like; or a C₂ to C₆ dialkylamino group such as dimethylamino group, diethylamino group, methylethylamino group and the like. R² represents hydrogen atom; a halogen atom such as chlorine atom, fluorine atom and the like; or a C₁ to C₅ alkoxy group such as methoxy group, ethoxy group, butoxy group and the like. R³ represents hydrogen atom, hydroxy group, -O-(CH₂)ₙ-COOH (wherein n represents an integer of from 1 to 5) such as -O-(CH₂)₂-COOH or -O-(CH₂)₃-COOH, or -O-CO-(CH₂)₁-COOH (wherein l represents an integer of from 1 to 3) such as -O-CO-(CH₂)₂-COOH or -O-CO-(CH₂)₃-COOH. R⁴ represents amino group or an amino group having 1 to 2 alkyl groups having 1 to 8 carbon atoms such as methylamino group, ethylamino group, butylamino group, hexylamino group, heptylamino group, dimethylamino group, diethylamino group, methylethylamino group and the like; or a 4 to 6 membered polymethyleneamino group which may be substituted by carboxyl group on the ring such as trimethyleneamino group, pentamethyleneamino group, 3-carboxypentamethyleneamino group and the like.

The preferable compounds used in the present invention among the compounds of the aforementioned formula (1) are shown in Table 1:

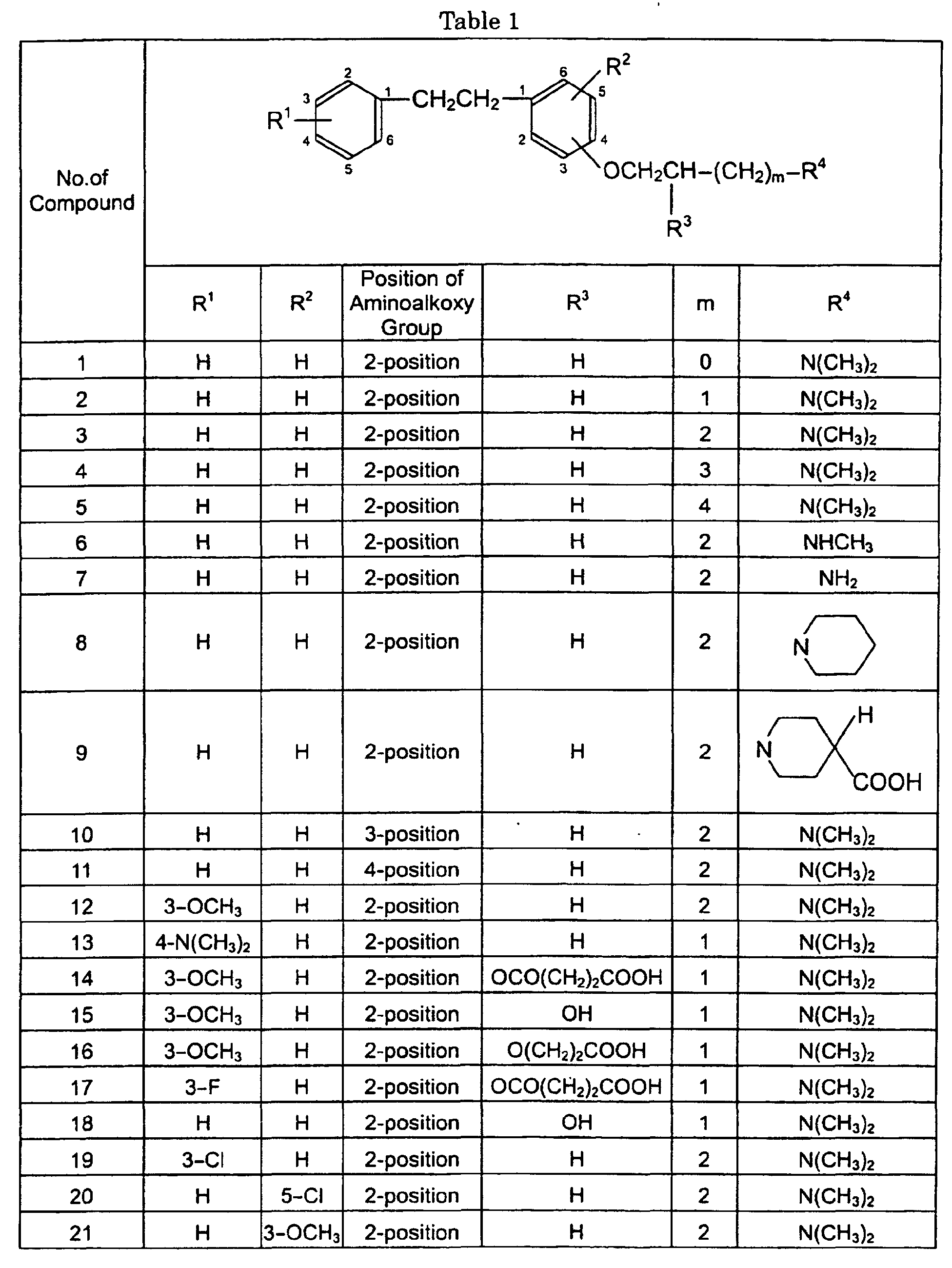

Particularly, the compounds wherein the aminoalkoxy group of -OCH₂C(R³)H-(CH₂)ₘ-R⁴ binds to the 2-position of the phenyl group are preferable. Preferable R¹ is hydrogen atom, a C₁ to C₅ alkoxy group or a C₂ to C₆ dialkylamino group, preferable R² is hydrogen atom, preferable R⁴ is an amino group having at least one alkyl group having 1 to 8 carbon atoms or a 4 to 6 membered polymethleneamino group of from trimethylene group to pentamethylene group, and m is an integer of 0 to 2.

Particularly preferred compounds are Compound No. 15 (hereinafter referred to as M-1) wherein R¹ is methoxy group, R² is hydrogen atom, R³ is hydroxyl group and R⁴ is dimethylamino group, and the corresponding succinic acid ester (Compound No. 14).

In the present invention, pharmaceutically acceptable salts of the compounds represented by the formula (1) can also be used. Such salts forming acids include, for example, hydrochloric acid, hydrobromic acid, sulfuric acid, phosphoric acid, nitric acid, acetic acid, succinic acid, adipic acid, propionic acid, tartaric acid, maleic acid, oxalic acid, citric acid, benzoic acid, toluenesulfonic acid, methanesulfonic acid and the like. Further, the solvates, for example hydrates of the compounds represented by the formula (1) and salts thereof can be used. Among them, the particularly preferable compound is (±)-1-[O-[2-(m-methoxyphenyl)ethyl]phenoxy]-3-(dimethylamino)-2-propyl hydrogensuccinate hydrochloride (optionally hereinafter referred to as sarpogrelate hydrochloride) represented by the following formula (4):

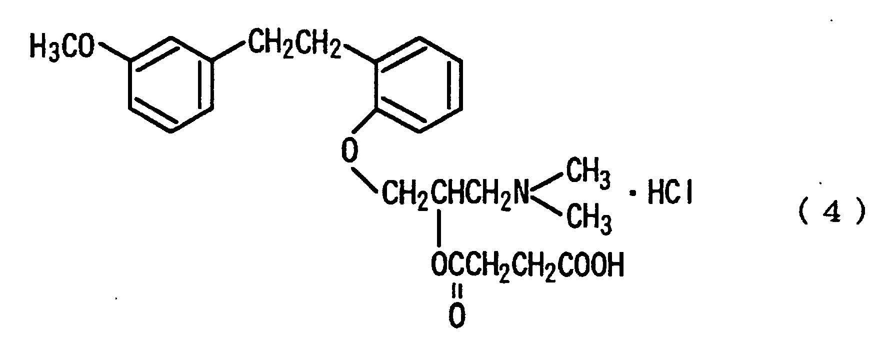

The aminoalkoxybibenzyls of formula (1) used in the present invention are known compounds as mentioned above, and can easily be synthesized in accordance with the methods described in Japanese Unexamined Patent Publication (Kokai) No. Sho 58-32847 or other similar methods thereto. The compound of the aforementioned formula (4) is sold as the brand name of Anplag (registered trade mark) and Anplag per se can be used in the present invention.

In the present invention, the aforementioned compounds use to the inhibition of thrombus/thrombogenesis. In the present invention, it has found that the aforementioned compounds are useful for the inhibition of thrombus/thrombogenesis in angina pectoris selected from chronic stable angina pectoris, unstable angina pectoris and the like, myocardial infarction, ischemic cerebrovascular failures such as TIA, cerebral infarction and the like. Further, in the present invention, the drugs of the present invention can be combinedly administered to the patients who already administered drugs selected from platelet antiaggregation agents, anticoagulants and brain circulation metabolic stimulants and the drugs of the present invention can be administered to the patients who administered the drugs selected from platelet antiaggregation agents, anticoagulants and cerebral circulation metabolism improving agents after discontinuation of the administration of said drugs.

The above mentioned platelet antiaggregation agents include an aspirin formulation (e.g. Bufferin or Bayaspirin), ticlopidine (e.g. Panaldine), cilostazol (e.g. Pletaal), dipyridamole (e.g. Persantin or Anginal), sodium ozagrel (e.g. Cataclot or Xanbon), ozagrel hydrochloride (e.g. Vega or Domenan), ethyl icosapentate (e.g. Epadel), beraprost sodium (e.g. Dorner or Procylin), an alprostadil formulation (e.g. Liple, Palux or Prostandin) and the like; anticoagulants include warfarin potassium (e.g. Warfarin), argatroban (e.g. Novastan or Slonnon), a heparin formulation (e.g. Fragmin or Heparin sodium) and the like; fibrin solubilizing agents include urokinase (e.g. urokinase), nasaruplase (e.g. Thrombolyse), alteplase (e.g. Grtpa), tisokinase (e.g. Plasvata), nateplase (Milyzer), pamiteplase (Solinase), monteplase (Cleactor) and the like. The brain circulation metabolic stimulants include citicoline (e.g. Ensign), adenosine triphosphate disodium (e.g.. Adetphos), cytochrome C (e.g. Cytochron-S), a combined formulation of cytochrome C (Cardiocrome), γ-aminobutyric acid (Gammalon), TRH (Hirtonin), calcium hopantenate (Hopate), meclofenoxate hydrochloride (Lucidril), amantadine hydrochloride (Symmetrel), tiapride hydrochloride (Gramalil), sulpride (e.g. Abilit or Dogmatyl), blood of young cattle-activated-substances (Solcoseryl), ifenprodil tartrate (Aponol or Cerocral), Vinpocetine (Caran), Nicergoline (Sermion), ibudilast (Ketas), dihydroergotoxine mesilate (e.g. Epos), nizofenone fumarate (Ekonal), fasudil hydrochloride (Eril) and the like.

The compounds represented by the aforementioned formula (1) are known to be efficacious for improving the intermittent claudication (Japanese Patent Unexamined Publication (Kokai) No. Hei 9-286722). Further, the combination with platelet antiaggregation agents, anticoagulants and cerebral circulation metabolic stimulants is possible for inhibition of thrombus/thrombogenesis as mentioned above. Accordingly, is can be easily thought that the drugs comprising as the active ingredients the compounds represented by the aforementioned formula (1) are combinedly used with the drugs selected from platelet antiaggregation agents other than said drugs, anticoagulants and cerebral circulation metabolic stimulants. The preferable platelet antiaggregation agents other than said drugs to be combinedly used include aspirin formulations, ticlopidine hydrochloride and the like and the preferable anticoagulants include warfarin potassium, urokinase and the like.

Any methods for administering the inhibitors of thrombus/thrombogenesis of the present invention can be employed.

Namely, parenteral administration such as subcutaneous injection, intraveneous injection, intramuscular injection, intraperitoneal injection and the like, and also oral administration can be applied.

The dose can be determined based on the age, condition of health, body weight of the patients, and, if treated simultaneously by other drugs, the type of such drugs, frequency of the treatment, the nature of the desired efficacy and the like.

In general, the daily dose of the active ingredient is 0.5 to 50 mg/kg of body weight, usually 1 to 30 mg/kg of body weight, and administered once or more.

For the oral administration, the compounds of the present invention are applied in the form of tablets, capsules, powders, liquid, elixir and the like, and for the parenteral administration, in the form of sterilized liquid forms such as liquids or suspensions or the like. When the above forms are applied, non-toxic pharmaceutical carries of solids or liquids can be contained in the composition.

Conventional gelatin-type capsule can be used as the example of the solid carrier. Further, the active ingredients can be used with or without auxiliaries to form tablets or package powders.

These capsules, tablets and powders generally contain 5 to 95%, preferably, 5 to 90% by weight of the active ingredient.

That is, these dosage forms contain 5 to 500 mg, preferably 25 to 250 mg of the active ingredient per dose.

Liquid carriers include water or oils originated from animals or vegetables such as petroleum, peanut oil, soybean oil, mineral oil, sesame oil and so on, or synthetic oils.

As the liquid carrier, saline, dextrose or similar sucrose, or glycols such as ethylene glycol, propylene glycol or polyethylene glycol are preferably used and particularly an injection using saline contains generally 0.5 to 20%, preferably, 1 to 10% by weight of the active ingredient.

### EXAMPLE

The present invention will be explained according to the following examples in more detail. However, the scope of the invention is not limited to these examples. In the following example, sarpogrelate hydrochloride used in the followings is applied Anplag (registered trademark) sold by Mitsubishi Pharma Corporation.

### Example 1

A 69-year-old female. She occurred transient cerebral ischemic attack (TIA). From three days after the onset, treatment with 160 mg/day of Cataclot (Ono Pharmaceutical Co., Ltd.) was started and it was continued for 2 weeks. Nine days after the completion of the administration of Cataclot (Ono Pharmaceutical Co., Ltd.), administration of 75 mg/day of sarpogrelate hydrochloride was started. The administration was continued for 337 days and during this term, neither recurrence of TIA nor onset of cerebral infarction was observed. Side effects also were not observed.

### Example 2

A 42-year-old male. He occurred TIA. He had not been treated with a drug after the onset, but administration of 150 mg/day of sarpogrelate hydrochloride was started from 20 days after the onset. The administration was continued for 337 days and during this term, neither recurrence of TIA nor onset of cerebral infarction was observed. Side effects also were not observed.

### Example 3

A 57-year-old female. She occurred TIA. From three days after the onset, treatment with 81 mg/day of Kids Bufferin (Lion Corporation) was started and this treatment was continued for about one month. The drug was then replaced with 300 mg/day of sarpogrelate hydrochloride and administration was continued for 337 days. During this term, neither recurrence of TIA nor onset of cerebral infarction was observed. Side effects also were not observed.

### Example 4

A 57-year-old male. He occurred cerebral infarction. From the day after the onset, he had been treated with 200 mg/day of Panaldine (Daiichi Pharmaceutical Co., Ltd.), 60 mg/day of Sabromin (Sankyo Co., Ltd.) and 600 mg/day of Comelian (Kowa Co., Ltd.). About 6 years after the onset of cerebral infarction, he occurred TIA and administration of the above-described three drugs was terminated. From 15 days after the onset of TIA, administration of 150 mg/day of sarpogrelate hydrochloride was started. It was continued for 337 days and during this term, neither recurrence of TIA nor onset of cerebral infarction was observed. Side effects also were not observed.

### Example 5

A 73-year-old female. She occurred cerebral infarction and, ten months after the occurrence of TIA. She had not been treated with a drug after the onset of TIA. From about one month after the onset of TIA, administration of 300 mg/day of sarpogrelate hydrochloride was started. It was continued for 337 days and during this term, recurrence of TIA and cerebral infarction was not observed. Side effects also were not observed.

### Example 6

A 73-year-old male. He occurred cerebral infarction. He had been treated with 200 mg/day of Panaldine (Daiichi Pharmaceutical Co., Ltd.), but administration of Panaldine (Daiichi Pharmaceutical Co., Ltd.) was stopped because he occurred TIA about 4 years and 7 months after the onset of cerebral infarction. On that day of the onset of TIA, the drug was replaced with 150 mg/day of sarpogrelate hydrochloride. The administration was continued for 337 days and during this term, recurrence of TIA and cerebral infarction was not observed. Side effects also were not observed.

### Example 7

A 68-year-old male. He occurred cerebral infarction. Although he had been treated with 300 mg/day of Panaldine (Daiichi Pharmaceutical Co., Ltd.), he occurred TIA three years after the onset of cerebral infarction. From that day of the onset of TIA, he was hospitalized for one week and subjected to intravenous drip containing 1 g of Nicholin H (Takeda Chemical Industries, Ltd.) and administration of 300 mg/day of Panaldine (Daiichi Pharmaceutical Co., Ltd.). The drug was then replaced with 75 mg/day of sarpogrelate hydrochloride and the administration was continued for 337 days. During this term, recurrence of TIA and cerebral infarction was not observed. Side effects also were not observed.

### Example 8

A 44-year-old male. He occurred cerebral infarction. He had been treated with 200 mg/day of Panaldine (Daiichi Pharmaceutical Co., Ltd.). He occurred TIA two years after the onset of cerebral infarction. From that day of the onset of TIA, administration of 200 mg/day of Panaldine (Daiichi Pharmaceutical Co., Ltd.) and, in addition, 162 mg/day of Kids Bufferin (Lion Corporation) was started. From about 2 months after the onset of TIA, the administration was replaced with that of 75 mg/day of sarpogrelate hydrochloride. The administration was continued for 337 days and during this term, recurrence of TIA and cerebral infarction was not observed. Side effects also were not observed.

### Example 9

A 73-year-old male. He occurred cerebral infarction. He had been treated with an antihypertensive and Warfarin (Eisai Co., Ltd.). From eight years after the onset, the drug was replaced with 300 mg/day of Panaldine (Daiichi Pharmaceutical Co., Ltd.), but about 10 months after the administration of Panaldine (Daiichi Pharmaceutical Co., Ltd.), cerebral infarction recurred. Immediately after the recurrence, he was treated with fluid for supplementation, urokinase and 300 mg/day of Panaldine (Daiichi Pharmaceutical Co., Ltd.). Administration of 300 mg of Panaldine (Daiichi Pharmaceutical Co., Ltd.) was continued for about 5 months after recurrence until it was replaced with the administration of 150 mg/day of sarpogrelate hydrochloride. The administration was continued for 496 days and during this term, recurrence of TIA and cerebral infarction was not observed. Side effects also were not observed.

### INDUSTRIAL APPLICABILITY

The present invention can provide thrombus/thrombogenesis inhibitors, particularly thrombus/thrombogenesis inhibitors found in ischemic cerebrovascular failures selected from transient cerebral ischemic attack and cerebral infarction

The present application was filed with claiming the conventional priority based on Japanese Patent Application No. 2001-292855.

## Claims

1. An inhibitor of thrombus/thrombogenesis which comprises as an active ingredient an aminoalkoxybibenzyl represented by the following formula (1) or a pharmaceutically acceptable salt thereof and a solvate thereof: 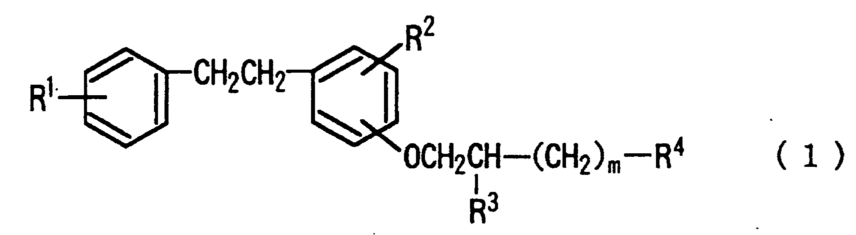 wherein R¹ represents hydrogen atom, a halogen atom, a C₁ to C₅ alkoxy group or a C₂ to C₆ dialkylamino group; R² represents hydrogen atom, a halogen atom or a C₁ to C₅ alkoxy group; R³ represents hydrogen atom, hydroxy group, -O-(CH₂)ₙ-COOH, wherein n represents an integer of from 1 to 5, or -O-CO-(CH₂)ₗ-COOH, wherein l represents an integer of from 1 to 3; R⁴ represents -N(R⁵)(R⁶), wherein R⁵ and R⁶ represent each independently hydrogen atom or a C₁ to C₈ alkyl group; or 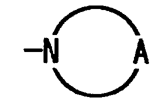 wherein A is a C₃ to C₅ alkylene group which may be substituted by carboxyl group; m represents an integer of from 0 to 5.

2. The inhibitor of thrombus/thrombogenesis according to Claim 1, wherein the compounds represented by the formula (1) is the compound selected from the compound represented by the formula (2), salts thereof and solvates thereof.

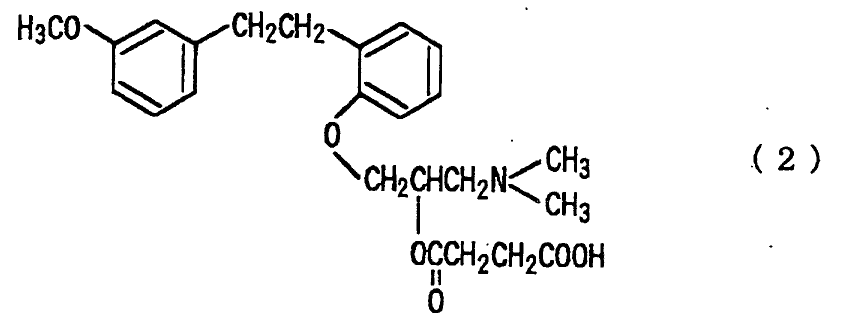

3. The inhibitor of thrombus/thrombogenesis according to Claim 2, wherein the salt is hydrochloride.

4. The inhibitor of thrombus/thrombogenesis according to any one of Claims 1 to 3, wherein the compound represented by the formula (1) is the compound selected the compounds represented by the following formula (3), salts thereof and solvates thereof.

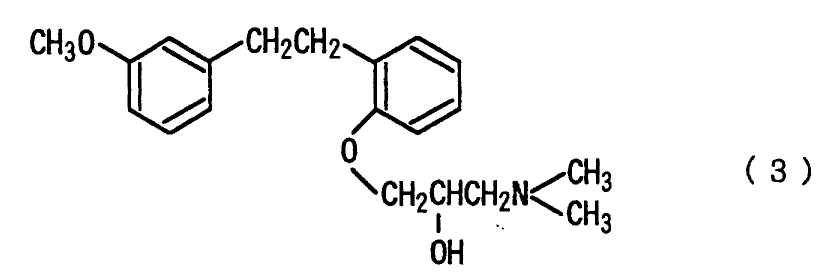

5. The inhibitor of thrombus/thrombogenesis according to any one of Claims 1 to 4, wherein the inhibition of thrombus/thrombogenesis is the inhibition of thrombus/thrombogenesis in the diseases selected from angina pectoris, and myocardial infaction and ischemic cerebrovascular failures.

6. The inhibitor of thrombus/thrombogenesis according to Claim 5, wherein the ischemic cerebrovascular failures are selected from transient cerebral ischemic attack and cerebral infarction.

7. The inhibitor of thrombus/thrombogenesis according to Claim 5, wherein angina pectoris is selected from transient chronic stable angina pectoris and unstable angina pectoris.

8. The inhibitor of thrombus/thrombogenesis according to any one of Claims 1 to 7, wherein said inhibitors are combinedly administered to the patients already administered the drugs selected from platelet antiaggregation agents, anticoagulants and brain circulation metabolic stimulants.

9. The inhibitor of thrombus/thrombogenesis according to any one of Claims 1 to 7, wherein said inhibitors are administered to the patients administered the drugs selected from platelet antiaggregation agents, anticoagulants and cerebral circulation metabolism improving agents after discontinuation of the administration of said drugs.

10. An improving agent for intermittent claudication which comprises administering drugs containing as the active ingredient aminoaloxybibenzyls represented by the following formula (1), pharmaceutically acceptable salts thereof and solvates thereof and drugs selected from platelet antiaggregation agents other than the aforementioned drugs, anticoagulants and cerebral circulation metabolism improving agents. 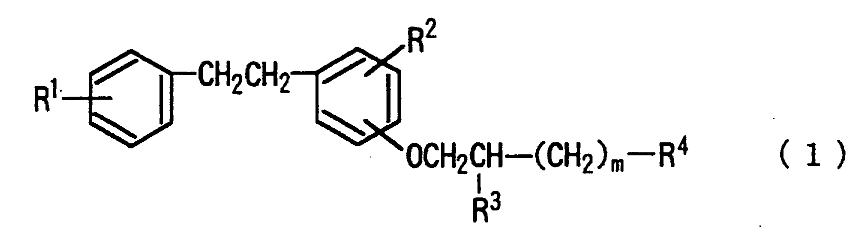 wherein R¹ represents hydrogen atom, a halogen atom, a C₁ to C₅ alkoxy group or a C₂ to C₆ dialkylamino group; R² represents hydrogen atom, a halogen atom or a C₁ to C₅ alkoxy group; R³ represents hydrogen atom, hydroxy group, -O-(CH₂)ₙ-COOH, wherein n represents an integer of from 1 to 5, or -O-CO-(CH₂)ₗ-COOH, wherein l represents an integer of from 1 to 3; R⁴ represents -N(R⁵)(R⁶), wherein R⁵ and R⁶ represent each independently hydrogen atom or a C₁ to C₈ alkyl group; or 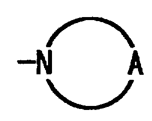 wherein A is a C₃ to C₅ alkylene group which may be substituted by carboxyl group; m represents an integer of from 0 to 5.

11. The improving agent for intermittent claudication according to Claim 10, wherein the compounds represented by the formula (1) is the compound selected from the compound represented by the following formula (2), salts thereof and solvates thereof.

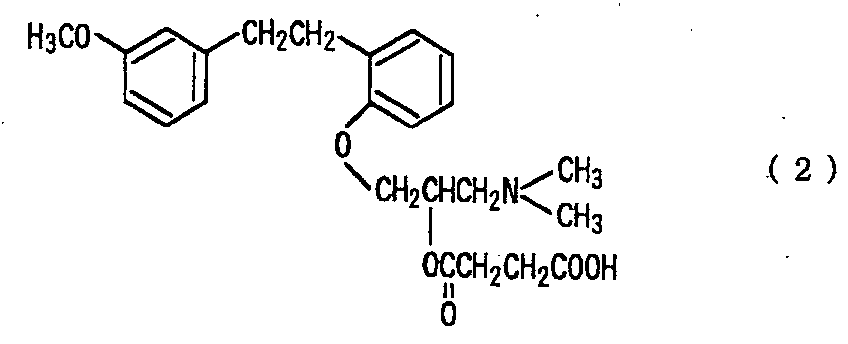

12. The improving agent for intermittent claudication according to Claim 11, wherein the salt is hydrochloride.

13. The improving agent for intermittent claudication according to any one of Claims 10 to 12, wherein the compound represented by the formula (1) is the compound selected the compounds represented by the following formula (3), salts thereof and solvates thereof.

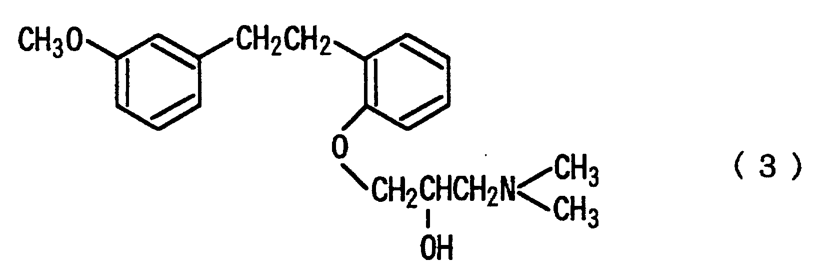

14. The improving agent for intermittent claudication according to any one of Claims 10 to 13, wherein the platelet antiaggregation agent is aspirin or ticlopidine hydrocholide.

15. The improving agent for intermittent claudication according to any one of Claims 10 to 13, wherein the anticoagulant is warfarin potassium or urokinase
